(19) Europäisches Patentamt
European Patent Office
Office européen des brevets

(11) EP 1 028 084 B1

(12) FASCICULE DE BREVET EUROPEEN

(45) Date de publication et mention
de la délivrance du brevet:
**02.07.2003 Bulletin 2003/27**

(51) Int Cl.⁷: **B66D 3/04**, B66D 3/18,
B66C 17/00

(21) Numéro de dépôt: **99420037.6**

(22) Date de dépôt: **12.02.1999**

(54) **Dispositif de levage pour le remplacement des anodes dans les cuves d'électrolyse pour la production d'aluminium**

Hebevorrichtung zur Ersetzung von Anoden in Elektrolyseöfen für die Aluminium-Herstellung

Hoisting device for the replacement of the anodes in the electrolytic cells for aluminium production

(84) Etats contractants désignés:
**DE ES FR GB GR IT NL SE**
Etats d'extension désignés:
**MK RO SI**

(43) Date de publication de la demande:
**16.08.2000 Bulletin 2000/33**

(73) Titulaires:
• **REEL S.A.**
**F-69450 Saint Cyr au Mont d'Or (FR)**
• **ASMI**
**59187 Dechy (FR)**

(72) Inventeur: **Piron, Gérard**
**69006 Lyon (FR)**

(74) Mandataire: **Vuillermoz, Bruno et al**
**Cabinet Laurent & Charras**
**B.P. 32**
**20, rue Louis Chirpaz**
**69131 Ecully Cédex (FR)**

(56) Documents cités:
**EP-A- 0 618 313     DE-A- 2 038 942
DE-A- 4 125 977     FR-A- 660 406
FR-A- 2 572 423     US-A- 2 189 447
US-A- 4 664 873     US-A- 4 886 174
US-A- 5 816 565**

## Description

La présente invention concerne de manière générale le changement des anodes usées au sein des cuves d'électrolyse de production d'aluminium. Elle concerne tout particulièrement un module susceptible de permettre l'extraction proprement dite des anodes usées et l'installation mettant en oeuvre de tels modules.

L'aluminium est produit industriellement par le procédé aujourd'hui bien connu de l'électrolyse ignée, c'est à dire mettant en oeuvre l'électrolyse de l'alumine dans un bain de cryolithe fondue selon la réaction :

$$Al_2O_3 + 2e^- + Na_3[AlF_6] \rightarrow 2\ Al + 3O_2 + 3C \rightarrow 3CO_2 + F$$

Alumine        cryolithe

Cette réaction, fortement exothermique, met donc un oeuvre un bain en fusion comprenant un mélange de cryolithe et d'alumine, dont la température est généralement supérieure à 800°C, et est fortement consommatrice d'électricité, de sorte que les installations fonctionnent en régime continu, afin de limiter les pertes d'énergie inhérentes aux phases de redémarrage.

Régulièrement, il convient de procéder au remplacement des différentes anodes, le plus souvent réalisées en carbone, au niveau de chacune des cuves, sans pour autant arrêter la réaction d'électrolyse.

De par le procédé mis en oeuvre, à savoir l'électrolyse ignée, il se forme à la surface supérieure du bain une croûte dure de cryolithe fluorée et d'alumine, cette croûte présentant l'avantage de conserver la chaleur au sein du bain, et donc de créer une enveloppe calorifuge.

Cependant, l'extraction des anodes usées hors du bain nécessite en première étape la rupture de cette croûte et, l'expérience montre que l'effort nécessaire pour arracher une anode usée de ladite croûte est sept à huit fois supérieur à la masse d'une anode neuve.

Par ailleurs, cet effort ne dure que sur quelques centimètres de course, alors que la course de levage de l'anode à chaque cycle est de l'ordre de trois à quatre mètres, typiquement 3,50 m.

A ce jour, dans les installations existantes, on a mis en oeuvre, afin d'effectuer ces opérations, des systèmes de vérins hydrauliques, qui à ce jour, présentent seuls un encombrement limité permettant leur intégration dans le volume disponible au dessus des cuves.

On a décrit dans le document US-A-5 816 565 un système de levage mettant en oeuvre deux organes de levage interagissant entre eux.

Cependant, quelle que soit la qualité de l'huile mise en oeuvre au niveau de ces vérins hydrauliques, compte tenu de la température élevée du bain de fusion, le risque d'incendie est permanent et, on souhaite s'affranchir de cet inconvénient rédhibitoire.

Les solutions proposées jusqu'alors pour éviter l'utilisation du système d'extraction hydraulique se heurtent à un problème d'encombrement et au génie civil des installations existantes. En effet, ces solutions, mettant typiquement en oeuvre un système à câble, demandent un encombrement largement supérieur, incompatible avec ces installations ou, nécessitant la réalisation de nouvelles installations, grevant de manière trop significative les coûts correspondants avant d'être susceptibles d'être amorties.

L'objet de l'invention est donc de proposer une installation pour le changement d'anodes usées au sein d'une série de cuves d'électrolyse, s'affranchissant de ces inconvénients, et notamment compatible avec les usines existantes et s'affranchissant des risques inhérents à la mise en oeuvre de dispositifs hydrauliques. Elle concerne en premier lieu un module porte-outil, destiné notamment à recevoir un outil pour permettre l'arrachage et le transfert des anodes usées. Ce porte-outil peut également intégrer tout type d'outil, et notamment un dispositif apte à rompre la croûte supérieure qui se forme à la surface du bain, mais également d'une pelle ou similaire, destinée à collecter les morceaux de croûte issus de cette rupture.

Ce module porte-outil, destiné à être mis en oeuvre au sein d'une installation de levage, est caractérisé en ce qu'il est muni de deux organes de levage motorisés de manière indépendante :

■ un premier organe, constitué d'un système de levage comprenant au moins un câble, un ou deux tambours motorisés électriquement, un mouflage au niveau duquel est fixé un porte-outil, ledit ou lesdits câbles venant s'enrouler sur ledit ou lesdits tambour(s) et étant renvoyé(s) au niveau du mouflage, ledit premier organe de levage étant destiné à assurer le déplacement d'une charge au niveau du mouflage à vitesse relativement importante et selon une course étendue ;
■ un second organe, dont l'un des éléments constitutifs est solidarisé à l'extrémité du ou des câbles dudit premier organe après renvoi au niveau du mouflage et comprenant des moyens permettant d'assurer en coopération avec ledit mouflage le déplacement vertical à vitesse réduite et selon une course limitée d'une charge plus importante.

L'invention consiste donc, dans un premier temps, à différencier deux mouvements, à savoir un mouvement nécessitant un effort intense sur une course courte à vitesse réduite, et un mouvement nécessitant un effort nettement plus réduit sur une course beaucoup plus longue, à une vitesse également beaucoup plus élevée.

Principalement, ce module est tout particulièrement approprié pour l'arrachage et le transfert des anodes usées. De fait, le second organe est destiné à permettre la mise en oeuvre d'un effort de traction intense, nécessaire et suffisant pour permettre l'arrachage proprement dit de l'anode usée hors du bain d'électrolyse, alors que le premier organe, après arrachage effectif de ladite anode, assure le déplacement de celle-ci en dehors de la cuve.

Selon une caractéristique avantageuse de l'invention, le premier organe est constitué par un système de levage par câble(s), portant un ou plusieurs câbles enroulés sur un ou plusieurs tambours mus par un moteur électrique, et munis d'un système de mouflage au niveau duquel est fixé un outil, par exemple de préhension de l'anode, de rupture de la croûte supérieure du bain d'électrolyse ou encore d'un outil faisant office de pelle de récupération des morceaux provenant de ladite couche.

Parallèlement, ledit second organe est constitué d'un vérin mécanique ou électro-mécanique, à l'extrémité duquel sont fixés le ou les câbles dudit premier organe.

En d'autres termes, l'invention consiste à réaliser un système de levage d'une charge muni d'un point fixe, ledit point fixe étant susceptible d'être rendu mobile sur une course limitée par le biais dudit second organe, c'est à dire dans le cas d'espèce, d'un vérin mécanique ou électro-mécanique.

Avantageusement, on munit le module d'un mou de câble, de telle sorte à limiter la charge appliquée par l'outil, dont est muni ledit module, sur un obstacle, et notamment en fond de cuve d'électrolyse. Par ailleurs, ce mou de câble permet de maintenir les spires de câbles parfaitement enroulées au niveau du ou des tambours, et est en outre conçu pour ne pas appliquer d'efforts au niveau du plan de pose, ainsi que précisé de manière plus détaillée ultérieurement.

Selon une autre caractéristique de l'invention, le module porte-outil comporte un mât de guidage vertical semi-rigide, solidarisé au châssis dudit module, et le long duquel coulisse un chariot porte-outil proprement dit. Ce faisant, on limite l'amplitude du débattement latéral du mouflage, notamment au voisinage du maximum de course potentielle autorisée par la longueur des câbles.

L'invention concerne également l'installation pour le changement d'anodes usées de cuves d'électrolyse de production d'aluminium.

Cette installation comprend un pont roulant susceptible de se déplacer au dessus desdites cuves et sur lequel se déplace selon une direction perpendiculaire à la translation du pont au moins un chariot muni d'un module porte-outils destiné à extraire et transférer les anodes usées hors des cuves, et à acheminer au niveau desdites cuves des anodes neuves.

Cette installation se caractérise en ce qu'elle comporte en outre deux autres chariots, également susceptibles de se déplacer sur le pont roulant selon une direction perpendiculaire au sens de déplacement du pont ;

- respectivement un premier chariot comportant un outil destiné à rompre la croûte superficielle qui se créée à la surface supérieure du bain d'électrolyse de chacune des cuves ;
- un second chariot comportant un outil muni d'une pelle, destiné à permettre la collecte de tout ou partie des morceaux provenant de la rupture de ladite croûte ;

les trois chariots étant susceptibles de se déplacer de manière indépendante les uns par rapport aux autres.

La manière dont l'invention peut être réalisée et les avantages qui en découlent ressortiront mieux de l'exemple de réalisation qui suit donné à titre indicatif mais non limitatif à l'appui des figures annexées.

La figure 1 est une représentation schématique en perspective de partie d'une usine pour la production d'aluminium montrant une série de cuves d'électrolyse

La figure 2 est une représentation schématique en perspective de l'installation conforme à l'invention, et montrant notamment le pont roulant muni de trois chariots distincts.

La figure 3 est une représentation schématique relative au principe de mise en oeuvre des modules porte-outils conformes à l'invention, dont la figure 4 est une représentation d'un exemple de système de câblage, et la figure 5 est une vue du dessus de la figure 4.

Les figures 6, 7 et 8 sont respectivement des représentations schématiques en perspective du module porte-outils conforme à l'invention, respectivement seul et associé avec deux modules de même nature, selon deux vues différentes.

Les figures 9 et 10 sont des représentations schématiques du fonctionnement du mou de câble mis en oeuvre au niveau dudit module conforme à l'invention.

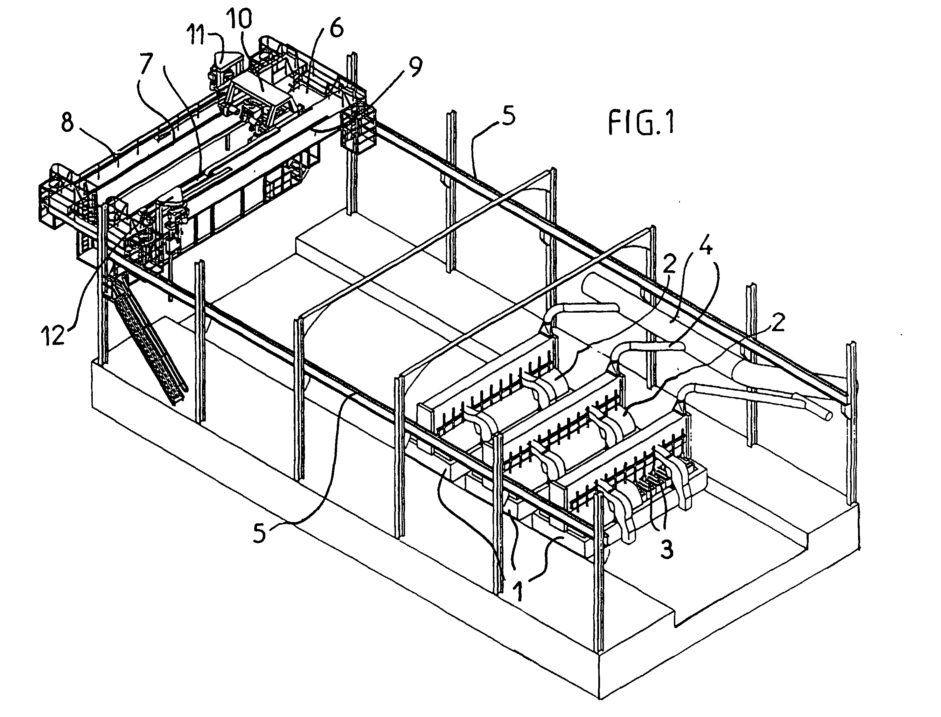

On a représenté au sein de la figure 1 une vue schématique d'une série de cuves d'électrolyse (1) pour la production d'aluminium, selon le procédé dit de l'électrolyse ignée, ces cuves étant installées au sein d'une usine susceptible de comporter plusieurs séries de telles cuves.

Comme on peut l'observer, l'installation comporte une pluralité de cuves (1), sensiblement identiques les unes aux autres, chacune des cuves comportant un capot d'accès (2) à l'intérieur de la cuve, et notamment au bain d'électrolyse en fusion proprement dit, susceptible de libérer ainsi l'accès, notamment aux anodes en carbone (3).

Compte tenu du dégagement de gaz fluorés, ainsi que de monoxyde et dioxyde de carbone, engendrés par la réaction d'électrolyse, chacune des cuves est fermée et est munie d'un dispositif de collecte des effluents gazeux sous la forme de tuyaux (4), lesdits effluents étant généralement retraités afin d'éviter leur rejet en l'état dans l'atmosphère.

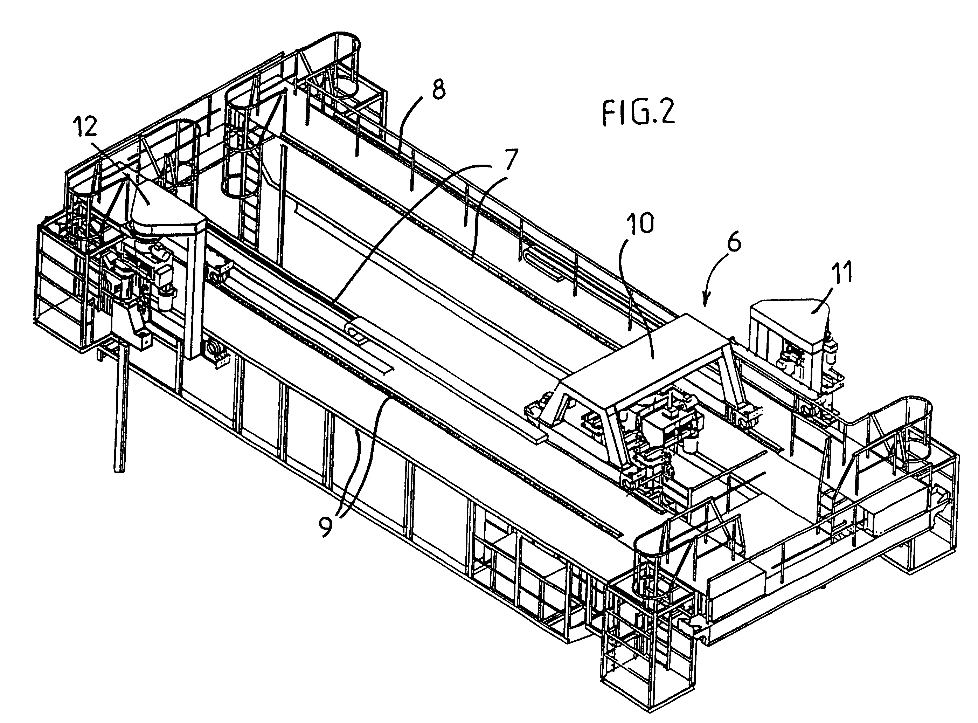

Le bâtiment renfermant cette série de cuves est équipés d'un chemin de roulement (5), sur lequel est susceptible de se déplacer un pont roulant (6), que l'on a représenté plus en détail au niveau de la figure 2.

Ce pont roulant comporte, selon l'invention, trois ensembles de chemins de roulement indépendants (7), (8) et (9), orientés perpendiculairement par rapport au chemin de roulement (5), et sur lesquels sont susceptibles de se déplacer des chariots, respectivement :

- (10) de support de modules d'acheminement des anodes neuves et d'évacuation des anodes usées, se déplaçant sur le chemin de roulement (7);
- (11) de support d'un outil de rupture de la croûte supérieure des bains d'électrolyse, se déplaçant sur le chemin de roulement (8);
- (12) de support d'un outil de collecte, typiquement une pelle, de tout ou partie des morceaux issus de la rupture de la croûte, se déplaçant sur le chemin de roulement (9).

Bien entendu, chacun de ces chariots est muni de son propre dispositif d'entraînement en direction, au moyen notamment de moteurs électriques.

Par ailleurs, ainsi qu'on peut bien l'observer sur la figure 2, les deux chariots externes respectivement portant les outils de rupture de croûte et la pelle, sont munis chacun de deux chemins de roulement, respectivement supérieur et inférieur, et donc des organes de coopération complémentaires, notamment des galets, propres à leur permettre un déplacement uniforme et guidé au niveau du pont.

La mise en oeuvre de cette installation s'avère donc tout particulièrement aisée : lorsqu'il convient de procéder à un changement d'une série d'anodes usées, un opérateur, présent au niveau de la cuve (1), procède à l'ouverture du capot correspondant (2) et un autre opérateur induit le déplacement du chariot (11) muni de l'organe de rupture de la croûte supérieure au lieu considéré. Pour ce faire, le pont roulant (6) est lui-même déplacé pour permettre la mise en place du chariot (11) à l'aplomb de la zone et de la cuve considérées.

Le chariot (12), muni du module portant la pelle est alors à son tour amené à l'aplomb de ladite cuve par mise en mouvement du pont roulant (6) et dudit chariot, de telle sorte à permettre l'introduction de la pelle au sein de celle-ci, afin de collecter les morceaux issus de la rupture de la croûte. Ces morceaux sont évacués dans une zone appropriée.

Enfin, le chariot de changement d'anodes (10) est à son tour amené à l'aplomb de la cuve, et notamment au lieu de changement des anodes, et un opérateur induit l'arrachage des anodes considérées et leur évacuation, également au niveau d'une zone de stockage définie, et la mise en place des nouvelles anodes.

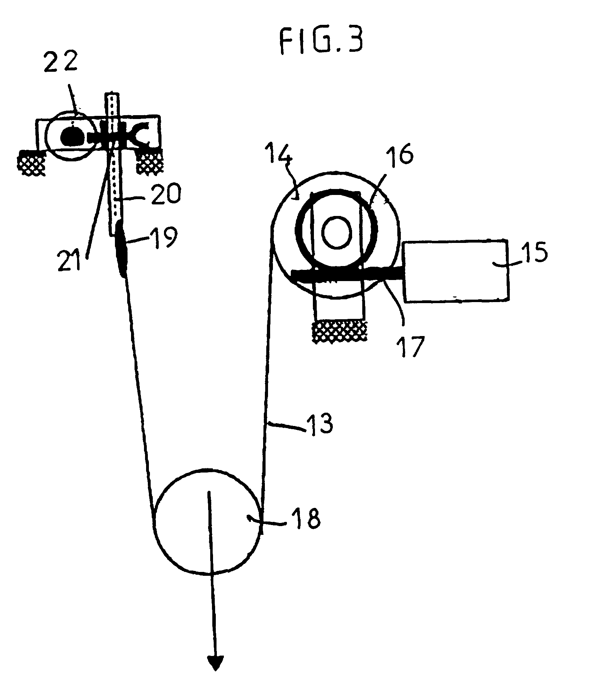

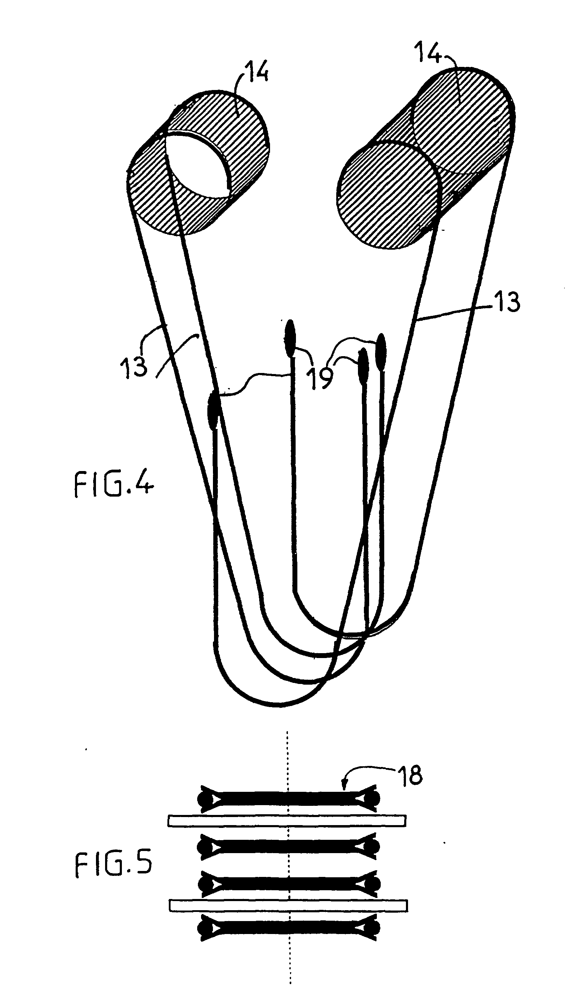

Il va être maintenant décrit plus en détail les modules porte-outils mis en oeuvre au sein de cette installation, et ce, en liaison avec les figures 3 à 10.

Selon une caractéristique fondamentale de l'invention, chacun des modules porte-outil, et notamment les modules de changement d'anodes, comportent deux systèmes d'activation indépendants. Ils comportent tout d'abord un système de câbles (13), enroulés sur un ou deux tambours (14), ces derniers étant mus en rotation au moyen d'un moteur électrique (15), par l'intermédiaire d'un réducteur à roue (16) et vis sans fin (17), chaque roue (16) étant solidaire et coaxiale à l'un des tambours (14) et venant coopérer avec la vis (17) colinéaire avec l'arbre moteur du moteur (15). Ces tambours sont positionnés au niveau du châssis supérieur de chacun des modules.

Le ou les câbles (13) viennent s'enrouler sur deux poulies constitutives d'un mouflage (18) et remontent en direction d'un point fixe (19).

La charge, et notamment le porte-outil considéré, est fixée au niveau du mouflage par tout moyen approprié, lui-même étant solidarisé à un chariot (25), ainsi que décrit plus en détail ci-après.

Selon l'invention, le point fixe (19) est lui-même solidarisé à l'extrémité de la vis (20) d'un vérin mécanique (21), mu par un moteur électrique (22).

Ainsi, ce double système de levage permet de mettre en oeuvre un levage classique assurant la course de déplacement des anodes sur des distances relativement importantes à une vitesse relativement élevée pour une charge réduite, et un levage avec arrachage, susceptible de développer un effort important sur une course réduite avec une vitesse limitée.

Ce dispositif permet par sa capacité de traction, d'une part d'assurer le développement de l'effort nécessaire à l'arrachage de l'anode usée, destinée à être remplacée, mais par ailleurs, compte tenu de la faible vitesse correspondante, il permet de faciliter la mise en place de l'anode neuve au niveau de la cuve.

Les organes de la chaîne cinématique, et notamment les roues (16), les tambours (14) et les câbles (13), sont dimensionnés en fonction de l'effort intense exercé par ledit second organe, et auquel ils sont soumis, compte tenu de leur sollicitation pendant la phase d'arrachage.

Par ailleurs, le choix d'un réducteur à roue et à vis (16, 17) au niveau du moteur grande vitesse assurant la rotation des tambours (14), permet, en utilisant le faible rendement indirect entre le réducteur et le tambour, de diminuer la taille des freins au niveau de l'arbre moteur dudit moteur (15), nécessaires lorsque l'on est en phase d'arrachage, et donc, de manière plus générale l'encombrement.

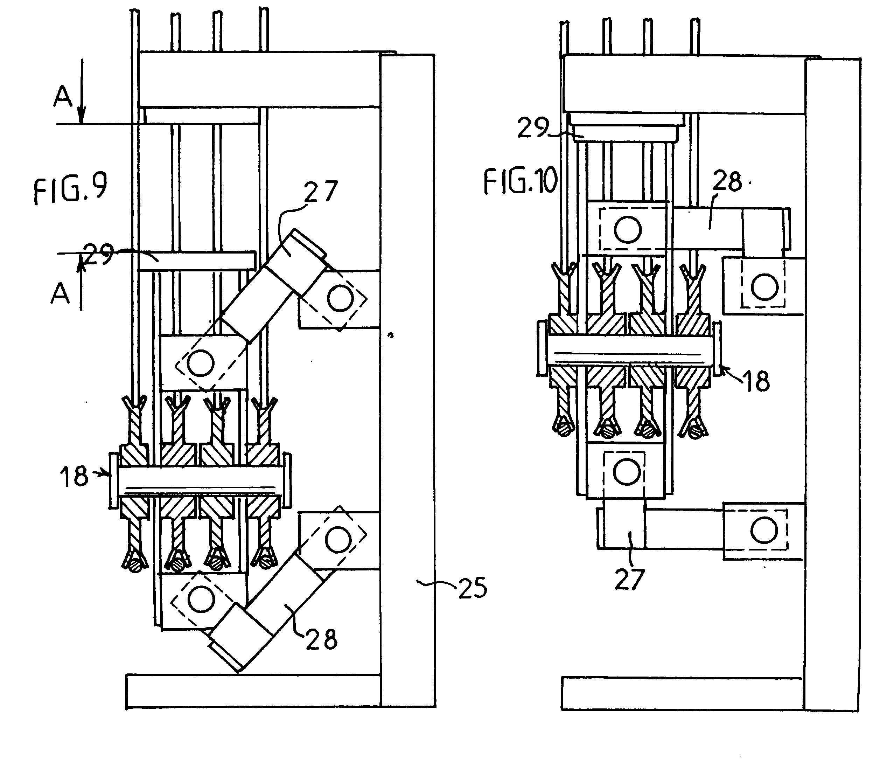

Avantageusement, on munit chacun des modules porte-outil de deux tambours (14) synchronisés par conception du réducteur, et recevant chacun deux câbles (13), de sorte que le nombre total de câbles est de quatre, ainsi que notamment représenté sur les figures 9 et 10, par exemple. De la sorte, il est possible de diminuer la taille des poulies, des tambours ainsi que celle des réducteurs, et de manière plus générale, de diminuer l'encombrement du dispositif.

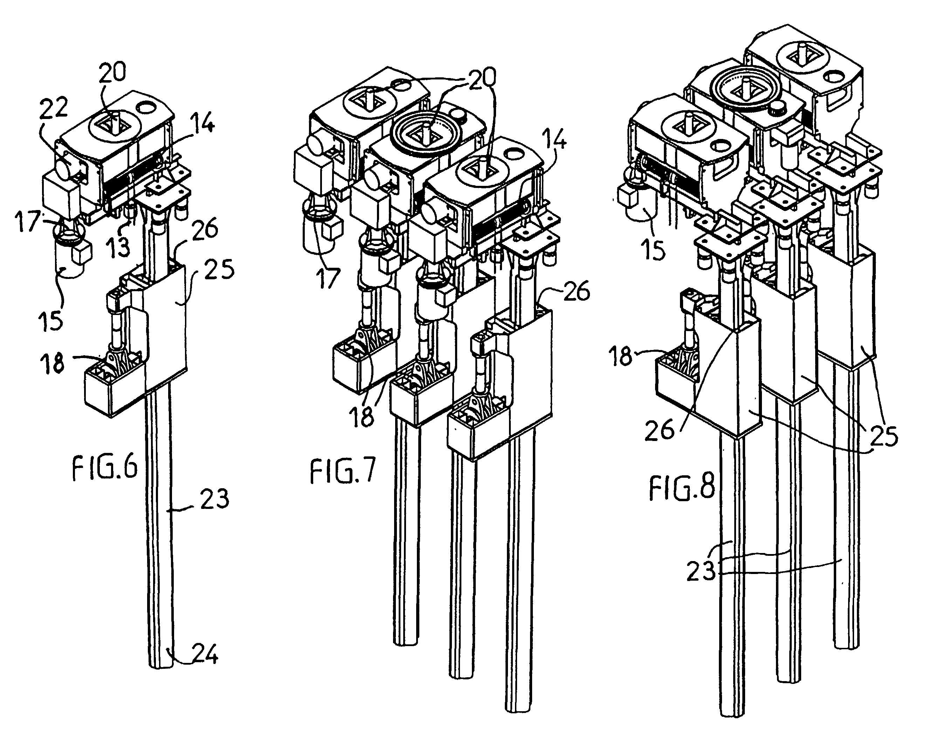

Selon une caractéristique avantageuse de l'invention, on associe les modules porte-outil de changement d'anodes par trois, ainsi que représentés sur les figures 7 et 8. Cette configuration est destinée à permettre le changement simultané de trois anodes usées. Il est bien entendu, néanmoins, que chacun des modules est susceptible de fonctionner indépendamment l'un de l'autre, de sorte que, nonobstant la mise en oeuvre d'un système à trois modules, un voire deux d'entre eux seulement peuvent être activés pour le changement d'anodes.

L'écartement entre les trois modules porte-outil correspond à l'entraxe des anodes. Cet écartement est susceptible d'être différent selon l'installation concernée et est donc réglable. Il est déterminé par des biellettes, dont la longueur correspond audit entraxe. Cette disposition permet l'adaptation du système à tout type d'installation.

Ainsi qu'on peut l'observer sur les figures 6 à 8, à chaque module porte-outil est associé un mât de guidage vertical (23), solidarisé au niveau du châssis supérieur du module par l'intermédiaire d'un dispositif de pré-contrainte incorporant des rotules, susceptible ainsi d'autoriser un certain degré de liberté au niveau de la partie inférieure (24) du mât. Ce mât (23) est destiné à permettre le guidage, notamment d'un chariot (25) contenant le mouflage (18) et par extension de la zone de suspension de la charge, ce chariot (25) étant muni de galets (26) prenant appui de part et d'autre dudit mât (23). Ce mât est semi-rigide. Le seuil de réglage de la pré-contrainte est tel, qu'on s'affranchit notablement des risques de débattement angulaire dudit porte-outil, notamment en bout de course, en cas d'efforts limités, et en particulier ceux inhérents au champ magnétique intense qui règne dans l'installation, et auquel sont donc soumis les éléments métalliques constitutifs d'une grande partie des éléments entrant dans la constitution du module.

Selon une autre caractéristique de l'invention, chaque module comporte un système de mou de câble, permettant ainsi de limiter le débattement vertical de l'outil lorsqu'il vient à entrer en interaction avec un obstacle, tel que par exemple lorsque la pelle vient à toucher le fond de la cuve. Dans ce dernier cas, on souhaite limiter au maximum un tel risque, compte tenu de la relative fragilité de la cuve. Par ailleurs, le mou de câble permet de maintenir parfaitement ordonnées les spires de câbles sur les tambours (14).

Ce mou de câble est plus particulièrement décrit en liaison avec les figures 9 et 10. Sa mise en oeuvre est obtenue en solidarisant de manière non fixe, le mouflage (18) au chariot (25) de déplacement du porte-outil sur le mât (23) au moyen d'un jeu de deux bielles (27, 28), susceptibles de se déplacer selon un parallélogramme déformable, et articulées respectivement sur le bâti (29) au niveau duquel est monté le mouflage, et sur ledit chariot (25), entre deux positions extrêmes, correspondant à la course de rattrapage de mou de câble représentée par la double flèche A sur la figure 9

Ainsi, la figure 9 représente le chariot (25) en appui sur un objet ou un obstacle environnant, le bâti (29) n'étant plus en contact avec ledit chariot (25). Un capteur (non représenté), positionné entre le chariot (25) et le bâti (29) aura donné l'ordre de l'arrêt du moteur de levage alors qu'il effectuait un mouvement de descente. La distance d'arrêt maximum du mouflage mobile est représentée par la double flèche A.

La figure 10 représente le chariot (25) suspendu sans contact avec l'environnement, le bâti (29) qui porte les poulies du mouflage étant en contact avec le chariot (25).

Avantageusement, on peut mettre en place un ressort entre la face supérieure du bâti (29) et le chariot (25), afin de diminuer le poids apparent dudit chariot sur un objet environnant, permettant dans cette hypothèse, de préserver la cuve d'électrolyse et son environnement.

Le module porte-outil conforme à l'invention est dimensionné pour accepter des efforts à la charge voisins de 10 tonnes pour des vitesses de l'ordre d'un millimètre par seconde. En revanche, pour des vitesses de l'ordre de 15 mètres par minute et des courses plus importantes, l'effort au déplacement est de l'ordre de 2 tonnes.

La mise en oeuvre de tels modules, activés par voie électrique, s'avère tout à fait adéquate pour les installations de production d'aluminium par électrolyse existantes, dans la mesure où, notamment, par la séparation physique des fonctions associées à des charges différentes, elle permet de limiter les réactions des galets sur les chemins de roulement des bâtiments existants, et par conséquent, elle ne nécessite aucune extension du génie civil, tout en permettant d'augmenter de manière significative les conditions de sécurité.

**Revendications**

1. Module porte-outil destiné à être mis en oeuvre au sein d'une installation de levage, comprenant deux organes de levage motorisés de manière indépendante :

   ■ un premier organe, constitué d'un système de levage comprenant au moins un câble (13), un ou deux tambours (14) motorisés électriquement, un mouflage (18) au niveau duquel est fixé un porte-outil, ledit ou lesdits câbles (13) venant s'enrouler sur ledit ou lesdits tambour(s) (14) et étant renvoyé(s) au niveau du mouflage (18), ledit premier organe de levage étant destiné à assurer le déplacement d'une charge au niveau du mouflage (18) à vitesse relativement importante et selon une course étendue ;
   ■ un second organe, dont l'un des éléments constitutifs est solidarisé à l'extrémité du ou des câbles (13) dudit premier organe après renvoi au niveau du mouflage (18), et comprenant des moyens permettant d'assurer en coopération avec ledit mouflage le déplacement vertical à vitesse réduite et selon une course limitée d'une charge plus importante.

2. Module porte-outil selon la revendication 1, destiné à réaliser l'arrachage d'anodes usées au sein d'une installation de production d'aluminium par électrolyse et le transfert desdites anodes et des anodes neuves venant en remplacement de ces dernières, *caractérisé* **en ce que** :

   ■ le premier organe est destiné à assurer le déplacement d'une anode à vitesse relativement importante et selon une course étendue ;
   ■ le second organe est destiné à assurer l'arrachage de ladite anode selon une vitesse réduite et pour une course limitée.

3. Module porte-outil selon l'une des revendications 1 et 2, *caractérisé* **en ce que** ledit second organe est constitué d'un vérin mécanique ou électro-mécanique (20, 21), à l'extrémité duquel sont fixés le ou les câbles (13) dudit premier organe, et destiné à exercer l'effort intense ou d'arrachage.

4. Module porte-outil selon l'une des revendications 1 à 3, *caractérisé* **en ce qu'**il est muni d'un mou de câble, destiné à limiter la charge appliquée par l'outil, dont est muni ledit module, sur un obstacle, et en particulier au fond d'une cuve à électrolyse.

5. Module porte-outil selon l'une des revendications 1 à 4, *caractérisé* **en ce qu'**il comporte un mât (23) de guidage vertical semi-rigide, solidarisé au châssis dudit module, et le long duquel est susceptible de coulisser un chariot (25) au niveau duquel est solidarisé le porte-outil proprement dit.

6. Module porte-outil selon la revendication 5, *caractérisé* **en ce que** le mât de guidage (23) est solidarisé au niveau du châssis du module par l'intermédiaire d'un dispositif de pré-contrainte, destiné à autoriser un certain degré de liberté au niveau de la partie inférieure (24) du mât.

7. Module porte-outil selon l'une des revendications 1 à 6, *caractérisé* **en ce que** la motorisation des tambours (14) est assurée par au moins un moteur électrique (15), couplé au tambours (14) par le biais d'un réducteur à roue (16) et à vis (17), chaque roue (16) étant solidaire et coaxiale à l'un des tambours (14) et venant coopérer avec la vis (17) colinéaire avec l'arbre moteur du moteur (15).

8. Module porte-outil selon la revendications 7, *caractérisé* **en ce que** les organes de la chaîne cinématique, et notamment les roues (16), les tambours (14) et les câbles (13), sont dimensionnés en fonction de l'effort intense exercé par ledit second organe (20, 21), et auquel ils sont soumis, compte tenu de leur sollicitation pendant la phase d'arrachage.

9. Installation pour le changement des anodes usées d'une usine de production d'aluminium par électrolyse ignée, comprenant un pont roulant (5) susceptible de se déplacer au dessus des cuves d'électrolyse (1), et sur lequel se déplace selon une direction perpendiculaire à la translation du pont au moins un chariot (10) muni d'un module porte-outil selon l'une des revendications 2 à 8, destiné à extraire et transférer les anodes usées hors des cuves, et à acheminer au niveau desdites cuves des anodes neuves, *caractérisée* **en ce qu'**elle comporte en outre deux autres chariots (11, 12), également susceptibles de se déplacer sur le pont roulant (5) selon une direction perpendiculaire à la translation dudit pont :

■ respectivement un premier chariot (11) comportant un outil destiné à permettre de rompre la croûte superficielle qui se créée à la surface supérieure du bain d'électrolyse de chacune des cuves (1);

■ un second chariot (12) comportant un outil muni d'une pelle, destiné à permettre la collecte de tout ou partie des morceaux provenant de la rupture de ladite croûte ;

les trois chariots étant susceptibles de se déplacer de manière indépendante les uns par rapport aux autres.

10. Installation pour le changement des anodes usées selon la revendication 9, *caractérisée* **en ce que** le chariot (10) est muni d'une pluralité de modules porte-outil, montés parallèlement les uns aux autres, et susceptibles de fonctionner concomitamment ou indépendamment les uns des autres.

11. Installation pour le changement des anodes usées selon la revendication 10, *caractérisée* **en ce que** l'écartement entre les modules porte-outil est réglable et correspond à l'entraxe des anodes, ledit écartement étant déterminé par des biellettes, dont la longueur correspond audit entraxe.

**Patentansprüche**

1. Werkzeugträger-Modul, welches dazu bestimmt ist, im Inneren einer Hebevorrichtung eingesetzt zu werden, und welches zwei unabhängig voneinander motorisierte Hebeorgane umfaßt:

• ein erstes Organ, bestehend aus einem Hebesystem mit mindestens einem Seil (13), einer oder zwei elektrisch motorisierten Trommeln (14), einem Seiltrieb (18), an dem ein Werkzeugträger befestigt ist, wobei das genannte oder die genannten Seil(e) (13) auf die genannte(n) Trommel(n) (14) aufgerollt werden und zu dem Seiltrieb (18) zurückgeleitet werden, wobei das genannte erste Hebeorgan dazu bestimmt ist, die Verlagerung einer Last im Bereich des Seiltriebs (18) mit einer relativ hohen Geschwindigkeit und entlang eines langen Weges sicherzustellen;
• ein zweites Organ, von dessen Bestandteilen einer am Ende des Seils oder der Seile (13) des genannten ersten Organs nach Zurückleiten zum Seiltrieb (18) fest verbunden ist und welches Mittel umfaßt, die es ermöglichen, in Zusammenwirkung mit dem genannten Seiltrieb die vertikale Verlagerung einer größeren Last bei verringerter Geschwindigkeit und entlang einem begrenzten Weg sicherzustellen.

2. Werkzeugträger-Modul nach Anspruch 1, welches dazu bestimmt ist, das Abziehen verbrauchter Anoden im Inneren einer Anlage für die Herstellung von Aluminium mittels Elektrolyse und den Transport der genannten Anoden und der neuen Anoden, die diese ersetzen sollen, durchzuführen, **dadurch gekennzeichnet, daß**

• das erste Organ dazu bestimmt ist, die Verlagerung einer Anode bei relativ hoher Geschwindigkeit und entlang einem langen Weg sicherzustellen;
• das zweite Organ dazu bestimmt ist, das Abziehen der genannten Anode mit einer verringerten Geschwindigkeit und über einen begrenzten Weg sicherzustellen.

3. Werkzeugträger-Modul nach einem der Ansprüche 1 und 2, **dadurch gekennzeichnet, daß** das genannte zweite Organ aus einem mechanischen oder elektromechanischen Stellantrieb (20,21) besteht, an dessen Ende das oder die Seil(e) (13) des genannten ersten Organs befestigt ist/sind und welcher dazu bestimmt ist, die starke oder abziehende Kraft auszuüben.

4. Werkzeugträger-Modul nach einem der Ansprüche 1 bis 3, **dadurch gekennzeichnet, daß** es mit einer Losestelle des Seils ausgestattet ist, welche dazu bestimmt ist, die durch das Werkzeug, mit dem das genannte Modul ausgestattet ist, auf ein Hindernis und insbesondere auf den Boden eines Elektrolysekessels aufgebrachte Last zu begrenzen.

5. Werkzeugträger-Modul nach einem der Ansprüche 1 bis 4, **dadurch gekennzeichnet, daß** es einen halbstarren vertikalen Führungsmast (23) aufweist, welcher mit dem Rahmen des genannten Moduls fest verbunden ist und entlang dem ein Schlitten (25) verschiebbar ist, an dem der Werkzeugträger selbst befestigt ist.

6. Werkzeugträger-Modul nach Anspruch 5, **dadurch gekennzeichnet, daß** der Führungsmast (23) an dem Rahmen des Moduls über eine Vorspannungs-Vorrichtung befestigt ist, welche dazu bestimmt ist, ein gewisses Spiel im Bereich des unteren Teils (24) des Mastes zuzulassen.

7. Werkzeugträger-Modul nach einem der Ansprüche 1 bis 6, **dadurch gekennzeichnet, daß** die Motorisierung der Trommeln (14) durch mindestens einen elektrischen Motor (15) sichergestellt ist, welcher mit den Trommeln (14) über ein Schneckenrad-(16) und Schnecken-(17)-Untersetzungsgetriebe gekoppelt ist, wobei jedes Schneckenrad (16) mit einer der Trommeln (14) fest verbunden und zu dieser koaxial ist und mit der Schnecke (17) zusammenwirkt, welche mit der Antriebswelle des Motors (15) kolinear ist.

8. Werkzeugträger-Modul nach Anspruch 7, **dadurch gekennzeichnet, daß** die Organe der kinematischen Kette, insbesondere die Schneckenräder (16), die Trommeln (14) und die Seile (13), in Abhängigkeit von der durch das genannte zweite Organ (20,21) ausgeübten und auf sie einwirkenden starken Kraft ausgelegt sind, unter Berücksichtigung ihrer Beanspruchung während der Phase des Abziehens.

9. Vorrichtung für den Austausch der verbrauchten Anoden einer Fabrik für die Herstellung von Aluminium durch Schmelzflußelektrolyse, welche eine über den Elektrolysekesseln (1) verschiebbare Rollbrücke (5) aufweist, auf der sich in einer zur Verschiebung der Brücke rechtwinkligen Richtung mindestens ein Schlitten (10) verschiebt, welcher mit einem Werkzeugträger-Modul nach einem der Ansprüche 2 bis 8 ausgestattet ist, welches dazu bestimmt ist, die verbrauchten Anoden aus den Kesseln herauszuziehen und zu befördern und den genannten Kesseln neue Anoden zuzuführen, **dadurch gekennzeichnet, daß** die Vorrichtung zudem zwei weitere Schlitten (11,12) aufweist, welche ebenfalls auf der Rollbrücke (5) in einer zur Verschiebung der genannten Brücke rechtwinkligen Richtung verschiebbar sind:

   • nämlich einen ersten Schlitten (11), welcher ein Werkzeug aufweist, das dazu bestimmt ist, das Aufbrechen der Oberflächenkruste, welche auf der oberen Oberfläche des Elektrolysebades jedes der Kessel (1) entsteht, zu ermöglichen;
   • und einen zweiten Schlitten (12), welcher ein mit einer Schaufel ausgestattetes Werkzeug aufweist, welches dazu bestimmt ist, das Einsammeln aller oder eines Teils der durch das Aufbrechen der genannten Kruste entstandenen Stücke zu ermöglichen;

   wobei die drei Schlitten voneinander unabhängig verschiebbar sind.

10. Vorrichtung für den Austausch der verbrauchten Anoden nach Anspruch 9, **dadurch gekennzeichnet, daß** der Schlitten (10) mit einer Mehrzahl Werkzeugträger-Module ausgestattet ist, welche parallel zueinander angeordnet sind und miteinander oder unabhängig voneinander funktionieren können.

11. Vorrichtung für den Austausch der verbrauchten Anoden nach Anspruch 10, **dadurch gekennzeichnet, daß** der Abstand zwischen den Werkzeugträger-Modulen einstellbar ist und dem Achsabstand der Anoden entspricht, wobei der genannte Abstand durch Schwingarme festgelegt ist, deren Länge dem genannten Achsabstand entspricht.

## Claims

1. Tool holder module intended to be used in lifting gear, equipped with two independently motorized lifting member:

   ■ a first member, which consists of a lifting system with at least one cable (13), one or two electrically motorized drum(s) (14), a sheaving system (18), to which is secured a tool holder, said cable(s) (13) being wrapped over said one or two drums(14) and being returned at said sheaving system (18), said first lifting member being intended to displace a load at the level of the sheaving system (18) at a relatively high speed and over a relatively large distance;
   ■ a second member, one of the constituent elements of which being secured to the end of the cable or cables (13) of said first member after return at the sheaving system (18), and comprising means which are intended, in cooperation with this system, to displace a heavier load vertically at a lower speed and over a limited distance.

2. Tool holder module according to claim 1, intended for pulling out spent anodes in a plant for producing aluminium by electrolysis and for transferring said anodes and fresh anodes which are to replace them, ***characterized* in that**:

   ■ said first member is intended to displace an anode at a relatively high speed and over a relatively large distance ;
   ■ said second member is intended to pull said anode out of the place in which it is inserted at a low speed and over a limited distance.

3.  Tool holder module according to one of Claims 1 and 2, *characterized* **in that** said second member consists of a mechanical or electro-mechanical jack (20, 21) to the end of which the cable or cables (13) of said first member are fixed, and which is intended to exert the intense or pulling-out force.

4.  Tool holder module according to one of Claims 1 to 3, *characterized* **in that** it has slack in the cable, so as to limit the load applied by the tool with which said module is fitted on an obstacle, and in particular at the bottom of an electrolysis tank.

5.  Tool holder module according to one of Claims 1 to 4, *characterized* **in that** it comprises a semi-rigid vertical guide post (23) secured to the chassis of said module, and along which a carriage (25) to which the tool holder proper is secured can slide.

6.  Tool holder module according to Claim 5, *characterized* **in that** the guide post (23) is secured to the module chassis via a preloading device intended to give the lower part (24) of the post a certain degree of freedom.

7.  Tool holder module according to one of Claims 1 to *6, characterized* **in that** the drums (14) are motorized by at least one electric motor (15) coupled to the drums (14) via a worm (17) and wheel (16) reduction gear set, each wheel (16) being secured to and coaxial with one of the drums (14) and cooperating with the worm (17) that is collinear with the motor drive shaft (15).

8.  Tool holder module according to Claim 7, *characterized* **in that** the members of the drive line, and in particular the wheels (16), the drums (14) and the cables (13) are dimensioned as a function of the intense force exerted by said second member (20, 21) and to which they are subjected, given the stress applied to them during the pulling-out phase.

9.  Installation for changing spent anodes in a works for producing aluminium by dry electrolysis, comprising a travelling crane (5) capable of moving over the electrolysis tanks (1), and on which there moves in a direction perpendicular to the translational movement of the crane at least one carriage (10) equipped with a tool holder module according to any one of claims 1 to 8, intended for extracting and transferring the spent anodes out of the tanks, and for bringing fresh anodes to said tanks, *characterized* **in that** this installation further comprises two more carriages (11, 12), also capable of moving on the travelling crane (5) in a direction perpendicular to the translational movement of said crane :

    •   respectively a first carriage (11) comprising a tool intended to allow the surface crust created at the upper surface of the electrolysis bath of each of the tanks (1) to be broken ;
    •   a second carriage (12) comprising a tool equipped with a shovel intended to allow all or some of the pieces resulting from breaking said crust to be collected;

    it being possible for the three carriages to move independently of one another.

10. Installation for changing spent anodes according to Claim 9, *characterized* **in that** the carriage (10) is equipped with a number of tool holder modules mounted in parallel with one another and capable of operating concomittantly or independently of one another.

11. Installation for changing spent anodes according to Claim 10, *characterized* **in that** the spacing between the tool holder modules is adjustable and corresponds to the distance between centres of the anodes, said spacing being determined by link rods, the length of which corresponds to said distance between centres.

FIG.1

FIG.2

# FIG.3

FIG.4

FIG.5

FIG.8

FIG.7

FIG.6

FIG.9

FIG.10

EP 1 028 084 B1